(19) Europäisches Patentamt
European Patent Office
Office européen des brevets

(11) **EP 2 887 861 B1**

(12) **EUROPÄISCHE PATENTSCHRIFT**

(45) Veröffentlichungstag und Bekanntmachung des Hinweises auf die Patenterteilung:
**20.11.2019 Patentblatt 2019/47**

(51) Int Cl.:
*A61B 5/00* *(2006.01)*     *A61N 1/36* *(2006.01)*
*A61B 5/0484* *(2006.01)*     *A61B 5/0488* *(2006.01)*
*A61N 1/05* *(2006.01)*

(21) Anmeldenummer: **13779728.8**

(22) Anmeldetag: **02.10.2013**

(86) Internationale Anmeldenummer:
**PCT/EP2013/002971**

(87) Internationale Veröffentlichungsnummer:
**WO 2014/053244 (10.04.2014 Gazette 2014/15)**

(54) **VORRICHTUNG ZUR UNTERSUCHUNG EINER PHASENVERTEILUNG ZUR ERMITTLUNG EINER KRANKHAFTEN INTERAKTION ZWISCHEN VERSCHIEDENEN HIRNAREALEN**

APPARATUS FOR EXAMINING A PHASE DISTRIBUTION TO IDENTIFY A PATHOLOGICAL INTERACTION BETWEEN DIFFERENT AREAS OF THE BRAIN

APPAREIL POUR ÉTUDIER UNE DISTRIBUTION DE PHASES POUR IDENTIFIER UNE INTERACTION PATHOLOGIQUE ENTRE DIFFÉRENTES RÉGIONS DU CERVEAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2012 DE 102012218057**

(43) Veröffentlichungstag der Anmeldung:
**01.07.2015 Patentblatt 2015/27**

(73) Patentinhaber: **Forschungszentrum Jülich GmbH 52425 Jülich (DE)**

(72) Erfinder: **TASS, Peter Alexander 52428 Jülich (DE)**

(74) Vertreter: **Müller-Boré & Partner Patentanwälte PartG mbB Friedenheimer Brücke 21 80639 München (DE)**

(56) Entgegenhaltungen:
**DE-A1-102010 016 461    US-A1- 2005 273 017
US-A1- 2009 054 800    US-A1- 2012 078 323
US-B1- 6 845 342**

• **MIRZA, A.F., MO, J., HOLT, J.L., KAIRALLA, J.A., HEFT, M.W., DING, M., AHN, A.H.: "Is There a Relationship between Throbbing Pain and Arterial Pulsations?", THE JOURNAL OF NEUROSCIENCE, Bd. 33, Nr. 22, 30. Mai 2012 (2012-05-30), Seiten 7572-7576, XP002720061,**

**Beschreibung**

Die vorliegende Erfindung betrifft eine Vorrichtung zur Untersuchung einer krankhaften Interaktion zwischen verschiedenen Hirnarealen.

Mehrere neurologische und psychiatrische Erkrankungen sind durch pathologisch gesteigerte Synchronisation von Neuronenpopulationen gekennzeichnet (vgl. z. B. "Pathological synchronization in Parkinson's disease: networks, models and treatments." von C. Hammond, H. Bergman und P. Brown, erschienen in Trends Neurosci. 30, 2007, Seiten 357 bis 364; "Tinnitus Perception and Distress Is Related to Abnormal Spontaneous Brain Activity as Measured by Magnetoencephalography" von N. Weisz, S. Moratti, M. Meinzer, K. Dohrmann und T. Elbert, erschienen in PLoS Med 2(6), 2005, Seiten 546 bis 553; "Imaging of Thalamocortical Dysrhythmia in Neuropsychiatry" von J. J. Schulman, R. Cancro, S. Lowe, F. Lu, K. D. Walton und R. R. Llinás, erschienen in Front. Hum. Neurosci. 5, 2011, Seite 69). In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Die pathologische Synchronisation von Neuronen äußert sich bei der Registrierung von Kollektiv-/Massen-/Makro-Signalen in einer erhöhten Amplitude der mittels Bandpassfilterung oder "Empirical Mode Decomposition" (vgl. z. B. "The empirical mode decomposition and the Hilbert spectrum for nonlinear and non-stationary time series analysis" von N. E. Huang, Z. Shen, S. R. Long, M. C. Wu, H. H. Shih, Q. Zheng, N.-C. Yen, C. C. Tung und H. H. Liu, erschienen in Proc. R. Soc. A: Math. Phys. Eng. Sci. 454, 1998, Seiten 903 bis 995; "Engineering analysis of biological variables: An example of blood pressure over 1 day" von W. Huang, Z. Shen, N. E. Huang und Y. C. Fung, erschienen in Proc. Nat. Acad. Sci. USA 95, 1998, Seiten 4816 bis 4821) gewonnenen Mode, welche zu dem oder den krankhaften Frequenzbereich(en) gehört; letzterer ist dem Fachmann bekannt (vgl. z. B. "Pathological synchronization in Parkinson's disease: networks, models and treatments." von C. Hammond, H. Bergman und P. Brown, erschienen in Trends Neurosci. 30, 2007, Seiten 357 bis 364; "Tinnitus Perception and Distress Is Related to Abnormal Spontaneous Brain Activity as Measured by Magnetoencephalography" von N. Weisz, S. Moretti, M. Meinzer, K. Dohrmann und T. Elbert, erschienen in PLoS Med 2(6), 2005, Seiten 546 bis 553; "Imaging of Thalamocortical Dysrhythmia in Neuropsychiatry" von J. J. Schulman, R. Cancro, S. Lowe, F. Lu, K. D. Walton und R. R. Llinäs, erschienen in Front. Hum. Neurosci. 5, 2011, Seite 69). Hierbei handelt es sich aber nicht um eine Alles-oder-Nichts-Gesetzmäßigkeit, d. h., auch Gesunde können in diesen speziellen Frequenzbereichen Leistungsdichte in den Leistungsdichtespektren, den sogenannten Powerspektren, aufweisen. Dementsprechend ermöglicht die Bestimmung der Leistungsdichtespektren derartiger z. B. MEG- oder EEG-Signale keine hinreichende Diskriminierung zwischen Gesunden und Patienten (vgl. z. B. "Imaging of Thalamocortical Dysrhythmia in Neuropsychiatry" von J. J. Schulman, R. Cancro, S. Lowe, F. Lu, K. D. Walton und R. R. Llinás, erschienen in Front. Hum. Neurosci. 5, 2011, Seite 69). Auch mittels Standard-Reizantworten (vgl. z. B. "A summation technique for the detection of small evoked potentials." von G. D. Dawson, erschienen in Electroencephalogr. Clin. Neurophysiol. 44, 1954, Seiten 153 bis 154; "Magnetoencephalography: Theory, instrumentation, and applications to noninvasive studies of the working human brain" von M. Hämäläinen, R. Hari, R. J. Ilmoniemi, J. Knuutila und O. V. Lounasmaa, erschienen in Rev. Mod. Phys., Band 65, 1993, Seiten 413 bis 497) kann diese Fragestellung nicht gelöst werden, d. h., nicht zwischen als pathologisch bzw. als nicht pathologisch zu wertender spektraler Leistungsdichte unterschieden werden.

Herkömmliche Vorrichtungen zur Untersuchung einer krankhaften Interaktion zwischen verschiedenen Hirnarealen sind in den Dokumenten US 2012/0078323 A1 und US 2005/0273017 A1 beschrieben.

Der Erfindung liegt die Aufgabe zugrunde, eine Vorrichtung anzugeben, die eine verlässliche elektrophysiologisch basierte Diagnose einer krankhaften Interaktion zwischen verschiedenen Hirnarealen ermöglichen. Insbesondere soll sich mit Hilfe der Vorrichtung eine Unterscheidung zwischen krankhafter und nicht-krankhafter spektraler Leistungsdichte in pathologischen Frequenzbereichen, die mit elektrophysiologischen Signalen, wie z. B. EEG-, MEG- oder EMG-Signalen gemessen wird, erzielen lassen. Insbesondere ist es Aufgabe der Erfindung, eine Diagnose einer krankhaften Interaktion zwischen den Hirnarealen zu ermöglichen, ohne dazu eine bivariate Analyse und Messung der Signale mindestens zweier interagierender Neuronenpopulationen zu benötigen.

Die der Erfindung zugrunde liegende Aufgabenstellung wird durch die Merkmale des Anspruchs 1 gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1    eine schematische Darstellung einer Vorrichtung zur Untersuchung einer krankhaften Interaktion zwischen verschiedenen Hirnarealen;

Fig. 2    eine schematische Darstellung einer Sequenz identischer Reize zur Untersuchung der krankhaften Interaktion;

Fig. 3A bis 3D    beispielhafte Darstellungen möglicher Verteilungen der normierten Phase eines Messsignals; und

Fig. 4 eine schematische Darstellung einer weiteren Vorrichtung zur Untersuchung einer krankhaften Interaktion zwischen verschiedenen Hirnarealen mittels akustischer Reize.

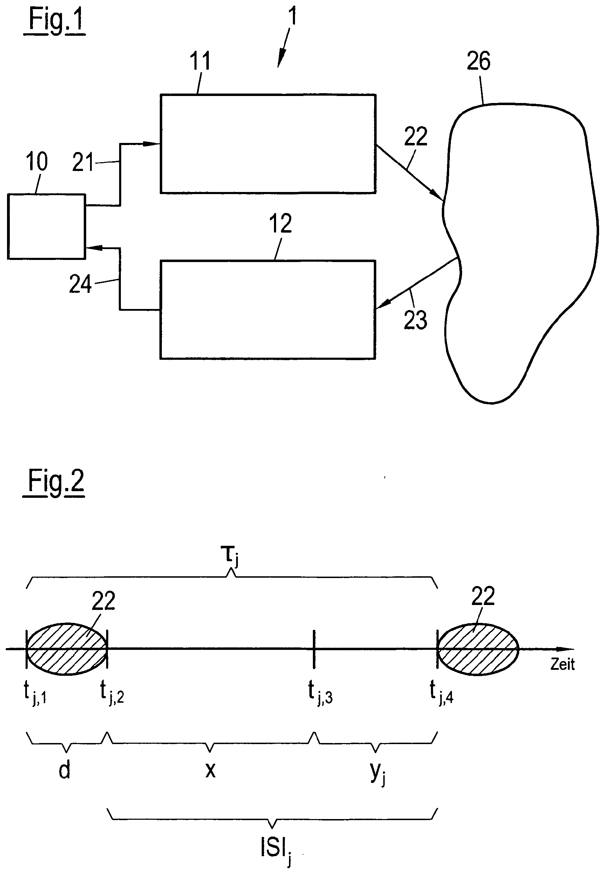

In Fig. 1 ist schematisch eine Vorrichtung 1 zur Untersuchung einer krankhaften Interaktion zwischen verschiedenen Hirnarealen dargestellt. Die Vorrichtung 1 besteht aus einer Steuer- und Analyseeinheit 10, einer Stimulationseinheit 11 und einer Messeinheit 12. Während des Betriebs der Vorrichtung 1 führt die Steuer- und Analyseeinheit 10 u. a. eine Steuerung der Stimulationseinheit 11 durch. Dazu erzeugt die Steuer- und Analyseeinheit 10 Steuersignale 21, die von der Stimulationseinheit 11 entgegengenommen werden. Die Stimulationseinheit 11 erzeugt anhand der Steuersignale 21 Reize 22, die einem Patienten verabreicht werden. Die Reize 22 werden dem Patienten als Sequenz identischer Einzelreize verabreicht und sind dazu ausgelegt, die Neuronen des Patienten in den zu untersuchenden Hirnarealen zu stimulieren.

Der durch die Reize 22 erzielte Stimulationseffekt wird mit Hilfe der Messeinheit 12 gemessen. Die Messeinheit 12 nimmt ein oder mehrere am Patienten gemessene Messsignale 23 auf, wandelt diese gegebenenfalls in elektrische Signale 24 um und führt diese der Steuer- und Analyseeinheit 10 zu. Insbesondere kann mittels der Messeinheit 12 die neuronale Aktivität in dem stimulierten Zielgebiet oder einem mit dem Zielgebiet eng verbundenen Gebiet des Gehirns 26 gemessen werden.

Die Steuer- und Analyseeinheit 10 verarbeitet die Signale 24, z. B. können die Signale 24 verstärkt und gefiltert werden, und analysiert die verarbeiteten Signale 24. Dabei untersucht die Steuer- und Analyseeinheit 10 die Verteilung der Phasen der von der Messeinheit 12 als Antwort auf die dem Patienten verabreichten Reize 22 aufgenommenen Messsignale 23 und ermittelt die Wahrscheinlichkeit, dass sich die Phasenverteilung von einer Gleichverteilung unterscheidet. Anhand dieser Analyse ermittelt die Steuer- und Analyseeinheit 10, ob eine krankhafte Interaktion zwischen den Hirnarealen vorliegt. Zur Durchführung ihrer Aufgaben kann die Steuer- und Analyseeinheit 10 beispielsweise einen Prozessor, z. B. einen Mikrocontroller, enthalten.

Die Reize 22 können Reize aus der Gruppe von akustischen, visuellen, taktilen, vibratorischen, propriozeptiven, thermischen, olfaktorischen und elektrischen transkutanen Reizen sein. Die Reize 22 können vom Patienten insbesondere bewusst wahrnehmbar sein. Die Stimulationseinheit 11 und insbesondere auch die Steuer- und Analyseeinheit 10 und die Messeinheit 12 sind bei dieser Ausgestaltung nicht-invasive Einheiten, d. h., während des Betriebs der Vorrichtung 1 befinden sie sich außerhalb des Körpers des Patienten und werden nicht operativ in den Körper des Patienten implantiert.

In einer alternativen Ausgestaltung wird die Stimulationseinheit 11 operativ in den Körper des Patienten implantiert und erzeugt anhand der Steuersignale 21 elektrische Reize 22, die dem Gehirn und/oder Rückenmark des Patienten verabreicht werden.

Die Messeinheit 12 enthält ein oder mehrere Sensoren, die es ermöglichen, mit hinreichender Zeitauflösung die neuronale Aktivität der stimulierten Neuronen zu detektieren. Als Sensoren können nicht-invasive Sensoren eingesetzt werden, z. B. Elektroenzephalographie (EEG)-Elektroden, Magnetenzephalographie (MEG)-Sensoren und Sensoren zur Messung lokaler Feldpotentiale (LFP). Die neuronale Aktivität kann auch indirekt durch Messung der damit einhergehenden Muskelaktivität mittels Elektromyographie (EMG)-Sensoren ermittelt werden.

Alternativ können die Sensoren in den Körper des Patienten implantiert sein. Als invasive Sensoren können beispielsweise epikortikale Elektroden, Tiefenhirnelektroden, sub- oder epidurale Hirnelektroden, subkutane EEG- oder EMG-Elektroden und sub- oder epidurale Rückenmarkselektroden dienen. Des Weiteren können an peripheren Nerven zu befestigende Elektroden als Sensoren eingesetzt werden.

Es kann durchaus vorgesehen sein, dass die einzelnen Komponenten der Vorrichtung 1, insbesondere die Steuer- und Analyseeinheit 10, die Stimulationseinheit 11 und/oder die Messeinheit 12, baulich voneinander getrennt sind. Die Vorrichtung 1 kann daher auch als System aufgefasst werden.

Die Vorrichtung 1 kann insbesondere zur Diagnose und Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, neuropathischem Schmerz, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden bzw. gekennzeichnet sein. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

Hirnareale mit einer synchronen und oszillatorischen neuronalen Aktivität interagieren zudem auf pathologisch übersteigerte Weise (vgl. z. B. "Mapping cortical hubs in tinnitus" von W. Schlee, N. Mueller, T. Hartmann, J. Keil, I. Lorenz und N. Weisz, erschienen in BMC Biol. 7, 2009, Seite 80).

Gemäß einer Ausgestaltung bewirken die dem Patienten verabreichten Reize 22 in der stimulierten Neuronenpopulation ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert gesetzt. Somit wird die Phase der neuronalen Aktivität der krankhaften Neuronenpopulation mittels einer gezielten Stimulation kontrolliert.

Im Folgenden wird die Funktionsweise der Vorrichtung 1 beschrieben. Ziel dabei ist die Detektion von diagnostisch relevanten, krankhaft gesteigerten Kopplungen zwischen Hirnarealen, in denen sich übermäßig synchronisierte, oszillatorische neuronale Aktivität findet.

Überraschenderweise hat sich gezeigt, dass simple Reize infolge der krankhaft gesteigerten Interaktion zwischen den Hirnarealen komplexe Reizantworten hervorrufen, die mit der Vorrichtung 1 nachweisbar sind. D. h., wird ein Reiz mehrfach appliziert, reagiert das bzw. reagieren die zugehörigen Hirnareale, bei akustischen Reizen z. B. der primäre und der sekundäre auditorische Cortex, nicht mit immer derselben, stereotypen Reizantwort. Vielmehr treten bei einem hinreichend großen Ensemble von identischen Einzelreizen 22, z. B. bei 50 oder 100 Einzelreizen 22, in einem oder mehreren Zeitintervallen nach Reizapplikation zwei oder mehrere Familien von Reizantworten auf, die sich bzgl. ihrer wechselseitigen Phasenlage unterscheiden. Z. B. können zwei Familien von Reizantworten zueinander gegenphasig sein, wodurch sich bei der standardmäßigen Berechnung einer gemittelten Reizantwort keine im Vergleich zum Zeitintervall vor der Reizapplikation signifikante Reizantwort ergibt, da sich die gegenphasigen Reizantworten herausmitteln.

Fig. 2 veranschaulicht eine mittels der Stimulationseinheit 11 durchgeführte Reizapplikation, bei der N identische Einzelreize 22 appliziert werden, wobei die Anzahl N beispielsweise größer 10 oder 50 oder 100 ist. Die Einzelreize 22 haben jeweils eine Dauer d und werden zu Zeitpunkten $t_{j,1}$ appliziert. Hierbei steht der Index j für den j-ten Reiz 22, wobei j = 1,2,...,N gilt. Der Index 1 steht für den Zeitpunkt des Beginns des j-ten Reizes 22. Der j-te Reiz 22 endet zum Zeitpunkt $t_{j,2}$, wobei d = $t_{j,2} - t_{j,1}$ gilt. Der nachfolgende j+1-te Reiz 22 beginnt zum Zeitpunkt $t_{j,4} = t_{j+1,1}$, wobei j = 1,2,...,N-1 gilt.

Das Zeitintervall zwischen dem Ende des j-ten Reizes 22 und dem Beginn des j+1-ten Reizes 22 wird als das auf den j-ten Reiz 22 folgende Inter-Stimulus-Intervall $ISI_j$ bezeichnet. Das Inter-Stimulus-Intervall $ISI_j$ kann von Reiz zu Reiz variieren, wobei es sich aus einem festen Anteil x und einem variablen Anteil $y_j$ zusammensetzt.

Die Dauer des gesamten Zeitintervalls, welches den j-ten Reiz 22 und das zugehörige Inter-Stimulus-Intervall $ISI_j$ enthält, beträgt $\tau_j = d + x + y_j$. Der feste Anteil x aller Inter-Stimulus-Intervalle $ISI_j$ soll so gewählt werden, dass frühe evozierte Antworten auf den j-ten Reiz 22 auf jeden Fall abgeklungen sind, bevor der j+1-te Reiz 22 appliziert wird. Bevorzugt wird deswegen 500 ms ≤ x ≤ 1000 ms gewählt, es können aber, z. B. zur Beschleunigung der Untersuchung, auch kleinere Werte, z. B. bis hinunter zu 300 ms, oder, z. B. zum Nachweis später komplexer Reizantworten, auch größere Werte, z. B. bis hinauf zu 3000 ms, gewählt werden.

Der variable Anteil yj wird für jedes Inter-Stimulus-Intervall $ISI_j$ bevorzugt zufällig und mit gleichverteilter Wahrscheinlichkeit aus einem Intervall $[0, y_{max}]$ gewählt. $y_{max}$ wird dabei so gewählt, dass es möglichst inkommensurabel ist, um Entrainment-Effekten, also Einschwing- bzw. Resonanzeffekten, durch eine Stimulation, deren Periode zur Periode der krankhaften oszillatorischen neuronalen Aktivität passt, vorzubeugen. Dies ist wichtig, um sicherzustellen, dass nur Nacheffekte, quasi Abklingeffekte, der Einzelreize 22 untersucht werden können. Ein hinreichend stark ausgeprägtes Entrainment kann die diagnostisch relevanten komplexen Reizantworten überdecken. Z. B. kann

$$y_{max} = \sqrt{2} \cdot 500$$ ms gewählt werden. Es können auch andere Werte wie $y_{max} = \sqrt{2} \cdot 1000$ ms oder

$$y_{max} = \sqrt{2} \cdot 300$$ ms gewählt werden. Wichtig bei der Wahl des Parameters $y_{max}$ ist, dass die mittlere Periode $\langle \tau_j \rangle = d + x + y_{max} / 2$ möglichst verschieden von der Periode der krankhaften oszillatorischen neuronalen Aktivität bzw. von ganzzahligen Vielfachen, insbesondere kleinen ganzzahligen Vielfachen davon ist. Auf diese Weise können Entrainment-Effekte vermieden werden.

Zeitgleich mit der Applikation der Einzelreize 22 werden von der Messeinheit 12 die Messsignale 23 aufgenommen und in Form der Signale 24 an die Steuerund Analyseeinheit 10 weitergeleitet. In der Steuer- und Analyseeinheit 10 kann eine Bandpassfilterung vorgenommen werden, um die für die jeweilige Krankheit relevanten Frequenzbänder herauszufiltern. Diese Frequenzbänder sind dem Fachmann bekannt. Z. B. findet sich bei Tinnituspatienten charakteristischerweise in tiefen Frequenzbereichen wie dem Deltaband im Bereich von 1 bis 4 Hz krankhaft übersteigerte neuronale oszillatorische Aktivität, die sich mittels Elektroenzephalographie (EEG) oder Magnetenzephalographie (MEG) nachweisen lässt (vgl. z. B. "Tinnitus Perception and Distress Is Related to Abnormal Spontaneous Brain Activity as Measured by Magnetoencephalography" von N. Weisz, S. Moratti, M. Meinzer, K. Dohrmann und T. Elbert, erschienen in PLoS Med 2(6), 2005, Seiten 546 bis 553).

Das Signal der k-ten Mode kann alternativ zu einer Bandpass-Filterung mit Hilfe des "Empirical Mode Decomposition"-Verfahrens berechnet werden (vgl. z. B. "The empirical mode decomposition and the Hilbert spectrum for nonlinear and non-stationary time series analysis" von N. E. Huang, Z. Shen, S. R. Long, M. C. Wu, H. H. Shih, Q. Zheng, N.-C. Yen, C. C. Tung und H. H. Liu, erschienen in Proc. R. Soc. A: Math. Phys. Eng. Sci. 454, 1998, Seiten 903 bis 995; "Engineering analysis of biological variables: An example of blood pressure over 1 day" von W. Huang, Z. Shen, N. E. Huang und Y. C. Fung, erschienen in Proc. Nat. Acad. Sci. USA 95, 1998, Seiten 4816 bis 4821). Das zeitabhängige Signal der k-ten Mode $s_k(t)$ lässt sich z. B. mittels der Hilbert-Transformation in die komplexe Zahlenebene transformieren und dort in die zugehörige zeitabhängige Amplitude $A_k(t)$ und die zeitabhängige Phase $\theta_k(t)$ zerlegen, wobei $s_k(t) = A_k(t) \cos[\theta_k(t)]$ gilt. Die Phasen $\theta_k(t)$ werden normiert

gemäß $\varphi_k(t) = \dfrac{\theta_k(t)}{2\pi} \bmod 1$, wobei k = 1,2,...,M gilt und M die Anzahl der Moden angibt.

Zur Analyse der von der Messeinheit 12 aufgenommenen Reizantworten wird an den Beginn $t_{j,1}$ eines jeden Reizes 22 ein Analysefenster [a,b] angeheftet, welches eine Zeitachse generiert: $t' \in [a,b]$, wobei in jedem Zeitfenster [a,b] der Beginn des zugehörigen Reizes 22 in t' = 0 liegt. Die maximal zulässige Breite des Fensters beträgt b - a = d + x, da es sonst zu Überlappungen der Fenster käme. Mit a = -x/2 und b = d + x/2 werden der prä- bzw. post-Stimulus-Bereich symmetrisch abgedeckt. Man kann durch die Wahl von a und b die Analyse bevorzugt auf den prä- bzw. post-Stimulus-Bereich fokussieren. Da die post-Stimulus-Dynamik, also die Reizantwort nach dem Ende des Reizes, von primärem Interesse ist, konzentrieren wir uns auf diese und wählen mit z. B. a = -x/4 und b = d + 3x/4 ein asymmetrisches Fenster, bei dem der kleinere prä-Stimulus-Bereich zur Bestimmung der prä-Stimulus-Messbasis verwendet wird, während der lange post-Stimulus-Bereich der Analyse der Reizantwort dient.

Um charakteristische Muster der Reizantworten der Phasen der der k-ten Mode $s_k$ zu bestimmen, betrachten wir die Verteilung der zugehörigen normierten Phase $\varphi_k$ zu den Zeiten t' relativ zum Reizbeginn, der in t' = 0 liegt. Diese Verteilung lautet $\{\varphi_k(t' + t_{j,1})\}_{j=1,...,N}$ mit

$t' \in [a,b]$.

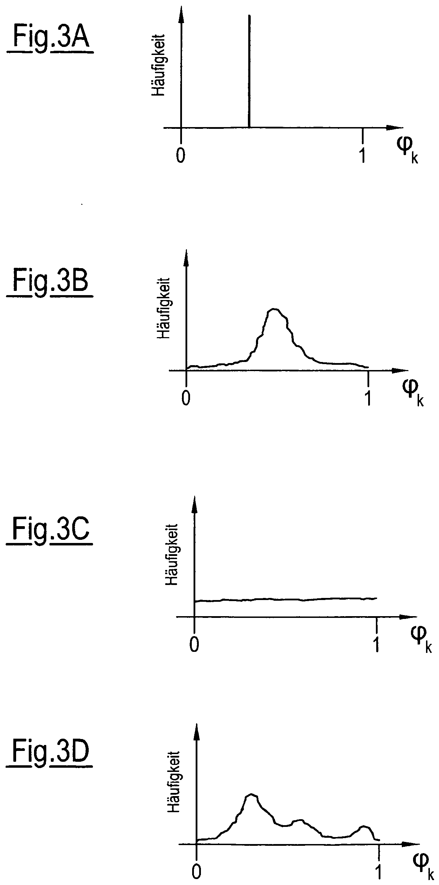

Eine zum Zeitpunkt t' zeitlich perfekt an den Reiz gekoppelte, also stereotype Reizantwort der normierten Phase $\varphi_k$ entspricht einer Dirac-artigen Verteilung zum Zeitpunkt t', bei der cpk idealerweise zum Zeitpunkt t' immer denselben Wert annimmt: $\{\varphi_k(t' + t_{1,1})\} = \{\varphi_k(t' + t_{j,1})\}$ für alle j = 2,3,...,N. Eine reizinduzierte ideale Phasenrücksetzung würde zu solchen Dirac-artige Verteilungen führen, wie sie beispielhaft in Fig. 3A dargestellt ist.

Typischerweise findet man unter realistischen Bedingungen, d. h. bei biologisch verträglichen Reizintensitäten und in Anwesenheit von Rauschkräften, im Falle einer reizinduzierten Phasenrücksetzung in einem bestimmten Teilintervall nach Reizbeginn einen Häufungswert, einen sogenannten Peak, in der Verteilung $\{\varphi_k(t' + t_{j,1})\}_{j=1,...,N}$. Beispielhaft ist ein solcher Peak in Fig. 3B gezeigt.

Falls der Reiz hingegen keinerlei Auswirkung auf die Phasendynamik auszuüben vermag, so ist die Verteilung $\{\varphi_k(t' + t_{j,1})\}_{j=1,...,N}$ nicht nur im prä-Stimulus-Bereich, sondern auch während des Reizes und im post-Stimulus-Bereich gleichförmig, wie beispielhaft in Fig. 3C gezeigt ist.

Wenn - z. B. in Abhängigkeit von der Phase zu Beginn der Stimulation - qualitativ unterschiedliche Reizantworten der Phase auftreten können, so hat die Verteilung $\{\varphi_k(t' + t_{j,1})\}_{j=1,...,N}$ zu bestimmten Zeiten während des Reizes und/oder im post-Stimulus-Bereich zwei oder mehr Peaks, wie Fig. 3D beispielhaft zeigt. Wenn es sich um zwei gegenphasig angeordnete Peaks ähnlicher Breite handelt, so mitteln sich die zugehörigen N Reizantworten raus, falls die Amplituden $A_k$ dieser beiden qualitativ unterschiedlichen Reizantworten nicht relevant voneinander abweichen.

Überraschenderweise zeigen krankhaft übersteigert gekoppelte Neuronenpopulationen charakteristischerweise komplexe Reizantworten der Phase der zugehörigen Messsignale, wie z. B. EEG-Signalen. D. h., nach der Reizapplikation treten Epochen auf, in denen die Verteilung $\{\varphi_k(t' + t_{j,1})\}_{j=1,...,N}$ signifikant von einer Gleichverteilung verschieden ist. Da es sich bei der insbesondere normierten Phase um eine periodische, zirkuläre Variable handelt, kann der sogenannte Kuiper-Test angewendet werden, der die zirkuläre Variante des Kolmogorov-Smirnov-Tests darstellt (vgl. z. B. "Tests concerning random points on a circle" von N. H. Kuiper, erschienen in Proceedings of the Koninklijke Nederlandse Akademie van Wetenschappen, Series A 63, 1960, Seiten 38 bis 47; "Circular Statistics in Biology" von E. Batschelet, Academic Press, London, 1981). Mit Hilfe des Kuiper-Tests lässt sich die Wahrscheinlichkeit $p_k(t)$ bestimmen, mit der sich die Verteilung $\{\varphi_k(t' + t_{j,1})\}_{j=1,...,N}$ zum Zeitpunkt t' von einer Gleichverteilung unterscheidet. Um schließlich zu bestimmen, ob ein Reiz eine signifikante Veränderung der Verteilung $\{\varphi_k(t' + t_{j,1})\}_{j=1,...,N}$ bewirkt, werden die prä-Stimulus-Verteilungen $\{p_k(t')\}_{t' \in [a,0[}$ betrachtet, aus der ein Schwellwert berechnet werden kann. Beispielsweise stiften die erste und 99-te Perzentile der Verteilung $\{p_k(t')\}_{t'\in[a,0[}$ ein Konfidenzintervall, wobei die 99-te Perzentile $\gamma_p$ als prä-Stimulus-Messbasis der entscheidende Wert ist: Übersteigt $p_k(t')$ nach Reizapplikation die 99-te Perzentile, liegt eine signifikante Abweichung von der Gleichverteilung vor.

Die durch die Stimulationseinheit 11 applizierten Reize 22 können so ausgestaltet sein, dass sie eine Phasenrücksetzung der oszillatorischen neuronalen Aktivität der stimulierten Neuronen bewirken. Bei primären sensorischen Arealen, die mit den zugehörigen Reizen stimuliert werden, also z. B. dem primären auditorischen Cortex bei der Stimulation mit akustischen Reizen, findet sich diese Phasenrücksetzung charakteristischerweise früh, also in enger zeitlicher Folge des Reizbeginns oder Reizendes (je nachdem, ob es sich um Reizantworten handelt, die von einem Reizbeginn oder einem Reizende ausgelöst werden), z. B. innerhalb der ersten 100 ms. Bei anderen, nicht sensorischen Hirnarealen kann die Phasenrücksetzung auch deutlich später, z. B. nach 200 ms auftreten. Die Phasenrücksetzung spiegelt primär die Auswirkung des Reizes auf ein Hirnareal dar. Die von der Vorrichtung 1 detektierten komplexen Reizantworten spiegeln jedoch die diagnostisch relevanten, krankhaft gesteigerten Kopplungen zwischen Hirnarealen wider. Deswegen sollten Zeitintervalle, in denen eine Phasenrücksetzung stattfindet, von der Analyse der komplexen Reizantworten ausgespart werden.

Hierzu wird die Differenz der Phasen vor und nach der Applikation des Reizes 22 bestimmt. Zuerst wird der zur normierten Phase $\varphi_k$ gehörende Index der Phasenrücksetzung                                   mittels

$$\sigma_k(t') = \left| \frac{1}{N} \sum_{j=1}^{N} \exp\left[i2\pi\varphi_k(t' + t_{j,1})\right] \right| \quad \text{be-}$$

stimmt. Es handelt sich hierbei um den Betrag des zirkulären Mittelwerts der Verteilung $\{\varphi_k(t' + t_{j,1})\}_{j=1,...,N}$ zum Zeitpunkt t'. Handelt es sich bei der Verteilung $\{\varphi_k(t' + t_{j,1})\}_{j=1,...,N}$ um eine Gleichverteilung, d. h., es liegt keine Phasenrücksetzung vor, bzw. um ein Dirac-artige Verteilung, d. h., es hat eine perfekte Phasenrücksetzung stattgefunden, so beträgt der Index der Phasenrücksetzung $\sigma_k(t') = 0$ bzw. $\sigma_k(t') = 1$.

Um zu bestimmen, ob ein Reiz eine signifikante Phasenrücksetzung bewirkt, werden die prä-Stimulus-Verteilungen $\{\sigma_k(t')\}_{t'\in[a,0[}$ betrachtet. Beispielsweise stiften die erste und 99-te Perzentile der Verteilung $\{\sigma_k(t')\}_{t'\in[a,0[}$ ein Konfidenzintervall, wobei auch hier wieder die 99-te Perzentile als prä-Stimulus-Messbasis der entscheidende Wert ist: Übersteigt $\sigma_k(t')$ nach der Applikation des Reizes 22 die 99-te Perzentile, liegt eine signifikante Abweichung von der Gleichverteilung vor. Infolge der Skalierungscharakteristik von $\sigma_k(t')$ ist es vorteilhaft, eine höhere Schwelle, z. B. das Vierfache der 99-ten Perzentile als prä-Stimulus-Messbasis, zur Detektion von Epochen mit Phasenrücksetzung anzuwenden. Diese Schwelle wird als $\gamma_\sigma$ bezeichnet.

Mit der Schwelle $\gamma_\sigma$ kann eine zeitabhängige Maskenfunktion $\mu(t')$ folgendermaßen berechnet werden:

$$\mu(t') = \begin{cases} 0 \text{ für } \sigma_k(t') \geq \gamma_\sigma \\ 1 \text{ für } \sigma_k(t') < \gamma_\sigma \end{cases}$$

Daraus ergibt sich die maskierte Wahrscheinlichkeit $P(t') = \mu(t')[p_k(t') - \gamma_p]$, welche die um die Epochen mit Phasenrücksetzung sowie um die prä-Stimulus-Messbasis korrigierte Wahrscheinlichkeit angibt, mit der zum Zeitpunkt t' eine komplexe Reizantwort, aber keine Phasenrücksetzung stattfindet. Die maskierte Wahrscheinlichkeit $P(t')$ wird im Intervall $]0,b]$ bestimmt. Eine Bestimmung im prä-Stimulus-Bereich liefert definitionsgemäß keine positiven Werte.

Die Steuer- und Analyseeinheit 10 kann verschiedene Informationen liefern. Beispielsweise kann die Steuer- und Analyseeinheit 10 derart ausgestaltet sein, dass sie mindestens einen Zeitpunkt t' detektiert, an dem für die maskierte Wahrscheinlichkeit $P(t') > 0$ gilt. Daraus kann auf das Vorliegen einer, wenn auch eventuell nur sehr kurzen Epoche mit einer komplexen Reizantwort geschlossen werden, die eine krankhaft gesteigerte Kopplung zwischen Hirnarealen widerspiegelt.

Ferner kann die integrale maskierte Wahrscheinlichkeit, also die über das Intervall $]0,b]$ integrierte maskierte Wahrscheinlichkeit, durch die Steuer- und Analyseeinheit 10 errechnet werden.

Außerdem ist es sinnvoll, die Gesamtdauer der einzelnen Epochen mit überschwelliger maskierter Wahrscheinlichkeit zu bestimmen. Die Gesamtdauer und die Ausprägungsstärke, also die integrale maskierte Wahrscheinlichkeit, sind die für die krankhaft gesteigerte Kopplung zwischen Hirnarealen relevanten Größen.

Diese Analyse wird durchgeführt für eine oder mehrere Moden von einem oder mehreren EEG-, MEG- oder LFP-Signalen oder sonstigen Messsignalen 23, die mit hinreichender Zeitauflösung die neuronale oszillatorische Aktivität repräsentieren.

In einer möglichen Ausführungsform verfügt die Vorrichtung 1 über Mittel, welche eine Visualisierung des Zeitgangs der maskierten Wahrscheinlichkeit ermöglichen.

Im Folgenden werden Reize 22 beschrieben, die sich für die Untersuchung einer krankhaften Interaktion zwischen verschiedenen Hirnarealen eignen. Zu beachten ist, dass das Auftreten der in dieser Anmeldung beschriebenen komplexen Reizantworten weniger von speziellen informationsverarbeitenden Eigenschaften eines Hirnareals abhängt, sondern vielmehr von der krankhaft starken Interaktion zwischen verschiedenen Hirnarealen hervorgerufen wird. Dementsprechend können auch Reizparameter und Reizarten verwendet werden, die sich von den im Folgenden beschriebenen Reizparametern und Reizarten deutlich unterscheiden.

Im Fall einer sensorischen Stimulation, d. h. bei der Applikation von insbesondere vibratorischen, taktilen, propriozeptiven, thermischen, visuellen oder olfaktorischen Reizen 22, werden die Reizparameter vorzugsweise derart gewählt, dass die Reize 22 ein evoziertes Potential auslösen. Besonders bevorzugt werden Reize 22 verwendet, die mehrere Eigenschaften bzw. Qualitäten enthalten. Z.B. können visuelle Reize 22 verwendet werden, die neben Helligkeitsinformation auch Kanteninformation und Farbinformation enthalten. Hierdurch werden unterschiedliche Hirnareale bzw. unterschiedliche Teilbereiche von Hirnarealen direkt stimuliert, wodurch die komplexen Reizantworten verstärkt auftreten. Bei einer invasiven Stimulation, d. h. einer elektrischen Stimulation des Gehirns oder Rückenmarks des Patienten, werden vorzugsweise Einzelpulse als Reize 22 appliziert. Ferner können Niederfrequenzpulszüge appliziert werden, wobei die Frequenz innerhalb des Pulszugs z. B. unter 50 Hz liegt.

Durch die erfindungsgemäße Vorrichtung werden - je nach Wahl der Reize - ein oder mehrere Hirnareale direkt stimuliert. Die hierdurch auftretenden komplexen Reizantworten sind charakteristisch für eine krankhaft gesteigerte Interaktion zwischen verschiedenen Hirnarealen. Die erfindungsgemäße Vorrichtung kann die krankhafte Interaktion selbst dann nachweisen, wenn nur ein Signal, das die neuronale Aktivität von nur einer Stelle des Gehirns repräsentiert, verwandt wird. Insbesondere müssen nicht mindestens zwei von unterschiedlichen Hirnarealen stammenden Signale gemessen werden und mit bivariaten Interaktionsanalysen analysiert werden. Die erfindungsgemäße Vorrichtung kann also eine krankhaft gesteigerte Interaktion mittels univariater Datenanalyse detektieren.

Zur Erzeugung akustischer Reize 22 können Einzeltöne oder Frequenzgemische mit einer Einhüllenden, z. B. einer Hanning-Window-Einhüllenden oder einer Cosinus-Einhüllenden, multipliziert werden. Als Beispiel seien Einzeltöne mit einer Dauer von 100 bis 300 ms und einer Reizstärke von 15 dB oberhalb der Hörschwelle genannt, die aus einem reinen Sinuston mit einer Hanning-Window-Einhüllenden hervorgegangen sind.

Die akustischen Reize 22 werden vom Patienten über ein oder beide Ohren aufgenommen, im Innenohr in Nervenimpulse umgesetzt und über den oder die Hörnerven an Neuronenpopulationen im Gehirn weitergeleitet. Die akustischen Reize 22 sind derart ausgestaltet, dass sie Neuronenpopulationen im auditorischen Cortex stimulieren. Durch die tonotope Anordnung des auditorischen Cortex wird bei der akustischen Stimulation des Innenohres mit einer bestimmten Frequenz ein bestimmter Teil des auditorischen Cortex aktiviert. Die tonotope Anordnung des auditorischen Cortex ist z. B. in den folgenden Artikeln beschrieben: "Tonotopic organization of the human auditory cortex as detected by BOLD-FMRI" von D. Bilecen, K. Scheffler, N. Schmid, K. Tschopp und J. Seelig (erschienen in Hearing Research 126, 1998, Seiten 19 bis 27), "Representation of lateralization and tonotopy in primary versus secondary human auditory cortex" von D. R. M. Langers, W. H. Backes und P. van Dijk (erschienen in NeuroImage 34, 2007, Seiten 264 bis 273) und "Reorganization of auditory cortex in tinnitus" von W. Mühlnickel, T. Elbert, E. Taub und H. Flor (erschienen in Proc. Natl. Acad. Sci. USA 95, 1998, Seiten 10340 bis 10343).

Einer Stimulation mit visuellen Reizen 22 kann eine Leuchtstärken- bzw. Helligkeitsvariation zugrunde liegen, beispielsweise können die Reize 22 als Pulse mit variierter Leuchtstärke bzw. Helligkeit appliziert werden. Als Beispiel, insbesondere für die Anwendung bei Migräne-Patienten, seien Schachbrettmuster-Umkehrreize genannt, die dem Patienten mittels eines Displays der Maße 22 x 22 cm mit jeweils 4 x 4 schwarzen bzw. weißen Quadraten mit einer Luminanz (Leuchtdichte) von jeweils 0,7 cd/m$^2$ und 117 cd/m$^2$ dargeboten werden.

Da unterschiedliche Stellen im Gesichtsfeld über die Linse des Auges auf unterschiedliche Stellen der Retina abgebildet werden und die unterschiedlichen Stellen der Retina über den Sehnerv wiederum mit unterschiedlichen Neuronen im Gehirn verbunden sind, können mit an unterschiedlichen räumlichen Orten angeordneten optischen Stimulationselementen gezielt unterschiedliche Neuronenpopulationen stimuliert werden. Die Zuordnung der Bereiche des Gesichtsfelds zu entsprechenden Bereichen des Gehirns ist beispielsweise in dem Artikel "Visual Field Maps in Human Cortex" von B. A. Wandell, S. O. Dumoulin und A. A. Brewer, erschienen in Neuron 56, Oktober 2007, Seiten 366 bis 383, beschrieben.

Vibratorische, taktile, propriozeptive, thermische, visuelle oder olfaktorische Reize 22 können dem Patienten mittels einer oder mehrerer geeigneter Stimulationseinheiten verabreicht werden, die auf die Haut aufgesetzt werden. Die Stimulationseinheiten können Stimulationselemente enthalten, die aus einer Ruhelage heraus auf die Hautoberfläche des Patienten geführt und eventuell in die Haut eingedrückt werden. Für die Applikation von thermischen Reizen können die Stimulationselemente eine entsprechende Temperatur aufweisen. Vibratorische, taktile, propriozeptive, visuelle und olfaktorische Reize 22 eignen sich insbesondere für Parkinson- und Dystonie-Patienten. Patienten mit neuropathischem Schmerz können neben vibratorischen, taktilen, propriozeptiven, visuellen und olfaktorischen Reizen 22 insbesondere mit thermischen Reizen behandelt werden.

Die gezielte Stimulation bestimmter Bereiche des Gehirns mittels vibratorischer, taktiler, propriozeptiver, thermischer und visueller Reize 22 wird durch die somatotope Zuordnung von Körperregionen zu diesen Bereichen ermöglicht. Beispielsweise können die Stimulationseinheiten am Fuß, Unterschenkel und Oberschenkel oder aber an der Hand, dem Unterarm und Oberarm des Patienten angebracht werden. Aufgrund der somatotopischen Gliederung der Nervenleitungsbahnen werden durch die an den jeweiligen Stellen applizierten Reize unterschiedliche Neuronen stimuliert. Die somatotope Zuordnung von Hautstellen zu Bereichen des Gehirns ist beispielsweise in A. Benninghoff et al.: "Lehrbuch der Anatomie des Menschen. Dargestellt unter Bevorzugung funktioneller Zusammenhänge. 3. Bd. Nervensystem, Haut und Sinnesorgane", Urban und Schwarzenberg, München 1964, beschrieben. Analoge Zusammenhänge gelten auch für das olfaktorische System.

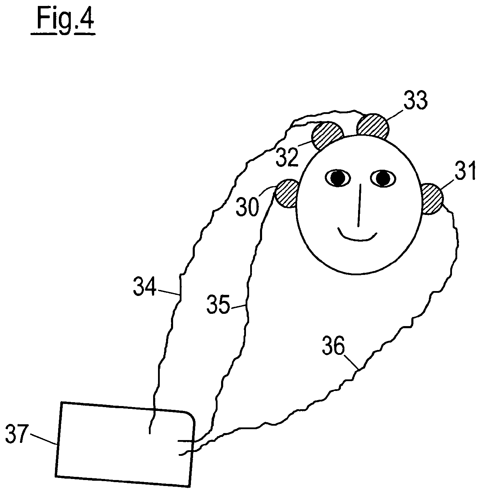

Fig. 4 zeigt schematisch ein Ausführungsbeispiel einer Vorrichtung zur Untersuchung einer krankhaften Interaktion zwischen verschiedenen Hirnarealen. Akustische Reize werden dem Patienten über Ohr- oder Kopfhörer 30, 31 verabreicht. Nicht-invasiv fixierte EEG-Elektroden 32, 33 als Messeinheit messen die EEG-Reizantworten. Kabel 34, 35, 36 verbinden die Ohr- oder Kopfhörer 30, 31 und die EEG-Elektroden 32, 33 mit einer Steuer- und Analyseeinheit 37, welche über Mittel zur Berechnung der maskierten Wahrscheinlichkeit und in einer möglichen Ausführungsform über Mittel zur Visualisierung des Zeitgangs der maskierten Wahrscheinlichkeit verfügt.

Bezogen auf den Anspruch 1 ist die Stimulationseinheit insbesondere derart ausgestaltet, dass sie identische Reize einem Patienten nacheinander verabreicht, wobei die Reize Neuronen des Patienten in den in zu untersuchenden Hirnarealen stimulieren und jeder der nacheinander verabreichten identischen Reize dieselben Neuronen stimuliert. Die Messeinheit nimmt Messsignale auf, die eine neuronale Aktivität der mit den identischen Reizen stimulierten Neuronen wiedergeben. Bei einer invasiven elektrischen Stimulation können dieselben Neuronen insbesondere dadurch stimuliert werden, indem die elektrischen Reize an derselben Stelle im Gehirn oder Rückenmark des Patienten appliziert werden. Taktile, vibratorische, propriozeptive, thermische und elektrisch transkutane Reize stimulieren dieselben Neuronen, wenn sie an derselben Stelle der Haut des Patienten appliziert werden. Olfaktorische Reize stimulieren dieselben Neuronen, wenn sie das olfaktorische System an derselben Stelle reizen. Visuelle Reize, die an derselben Stelle im Gesichtsfeld des Patienten erzeugt werden, stimulieren ebenfalls dieselben Neuronen. Um mit akustischen Reizen dieselben Neuronen zu stimulieren, können die Reize dieselbe Frequenz bzw. dieselben Frequenzen aufweisen.

## Patentansprüche

1. Vorrichtung (1) zur Untersuchung einer krankhaften Interaktion zwischen verschiedenen Hirnarealen, umfassend

   eine Stimulationseinheit (11), die derart ausgestaltet ist, dass sie identische Reize einem Patienten nacheinander verabreicht, wobei die Reize Neuronen des Patienten in den zu untersuchenden Hirnarealen stimulieren,

   eine Messeinheit (12) zum Aufnehmen von Messsignalen, die eine neuronale Aktivität der stimulierten Neuronen an nur einem Hirnareal des Gehirns wiedergeben, und

   eine Steuer- und Analyseeinheit (10) zur Steuerung der Stimulationseinheit und zur Analyse der Messsignale, wobei die Steuer- und Analyseeinheit (10) derart ausgestaltet ist, dass sie die Stimulationseinheit (11) derart ansteuert, dass diese dem Patienten die Reize verabreicht, und

   die Messsignale in die komplexe Zahlenebene transformiert, die Verteilung der Phasen der von der Messeinheit (12) als Antwort auf die dem Patienten verabreichten Reize aufgenommenen Messsignale in der komplexen Zahlenebene untersucht und anhand der Verteilung der Phasen der Messsignale, die die neuronale Aktivität der stimulierten Neuronen an nur dem einen Hirnareal des Gehirns wiedergeben, die Wahrscheinlichkeit ermittelt, mit der sich die Phasenverteilung von einer Gleichverteilung unterscheidet, um zu bestimmen, ob eine krankhafte Interaktion zwischen den Hirnarealen vorliegt.

2. Vorrichtung (1) nach Anspruch 1, wobei die Messeinheit (12) EEG-Elektroden, MEG-Sensoren, EMG-Sensoren, LFP-Sensoren und/oder implantierbare Sensoren umfasst.

3. Vorrichtung (1) nach Anspruch 1 oder 2, wobei die Stimulationseinheit (11) nicht-invasiv ist und derart ausgestaltet ist, dass sie Reize aus der Gruppe der akustischen, visuellen, taktilen, vibratorischen, propriozeptiven, thermischen, olfaktorische und elektrischen transkutanen Reize generiert.

4. Vorrichtung (1) nach Anspruch 1 oder 2, wobei die Stimulationseinheit (11) ein oder mehrere implantierbare Elektroden zur Verabreichung elektrischer Reize umfasst.

5. Vorrichtung (1) nach einem der vorhergehenden Ansprüche, wobei die Steuer- und Analyseeinheit (10) die Stimulationseinheit (11) derart ansteuert, dass die Abstände zwischen aufeinander folgenden Reizen variiert werden.

6. Vorrichtung (1) nach einem der vorhergehenden Ansprüche, wobei die Steuer- und Analyseeinheit (10) derart ausgestaltet ist, dass sie aus den vor der Verabreichung eines Reizes aufgenommenen Messsignalen einen Schwellwert ermittelt und die aus den nach der Verabreichung des Reizes aufgenommenen Messsignalen ermittelte Wahrscheinlichkeit, mit der sich die Phasenverteilung von einer Gleichverteilung unterscheidet, mit dem Schwellwert vergleicht, um zu bestimmen, ob eine krankhafte Interaktion zwischen den Hirnarealen vorliegt.

**7.** Vorrichtung (1) nach einem der vorhergehenden Ansprüche, wobei die Steuer- und Analyseeinheit (10) derart ausgestaltet ist, dass sie zur Ermittlung der Wahrscheinlichkeit, mit der sich die Phasenverteilung von einer Gleichverteilung unterscheidet, den Kuiper-Test einsetzt.

**8.** Vorrichtung (1) nach einem der vorhergehenden Ansprüche, wobei die Steuer- und Analyseeinheit (10) derart ausgestaltet ist, dass sie

mindestens einen Zeitpunkt detektiert, an dem die Wahrscheinlichkeit, mit der sich die Phasenverteilung von einer Gleichverteilung unterscheidet, über einem vorgegebenen Schwellwert liegt, und/oder

die Wahrscheinlichkeit, mit der sich die Phasenverteilung von einer Gleichverteilung unterscheidet, über einen vorgegebenen Zeitraum integriert, und/oder

die Gesamtdauer von Zeiträumen ermittelt, in denen die Wahrscheinlichkeit, mit der sich die Phasenverteilung von einer Gleichverteilung unterscheidet, über einem vorgegebenen Schwellwert liegt.

**Claims**

**1.** An apparatus (1) for examining a pathological interaction between different brain areas, comprising a stimulation unit (11) which is configured such that it successively administers identical stimuli to a patient, wherein the stimuli stimulate neurons of the patient in the brain areas to be examined; a measuring unit (12) for recording measured signals which reproduce a neural activity of the stimulated neurons at only one brain area of the brain; and a control and analysis unit (10) for controlling the stimulation unit and for analyzing the measured signals, wherein the control and analysis unit (10) is configured such that it controls the stimulation unit (11) such that it administers the stimuli to the patient; and transforms the measured signals into the complex plane, examines the distribution of the phases of the measured signals recorded by the measuring unit (12) in the complex number plane as a response to the stimuli administered to the patient and determines the probability with which the phase distribution differs from an equal distribution on the basis of the distribution of the phases of the measured signals that reproduce the neural activity of the stimulated neurons at only the one brain area of the brain in order to determine whether a pathological interaction between the brain areas is present.

**2.** An apparatus (1) in accordance with claim 1, wherein the measuring unit (12) comprises EEG electrodes, MEG sensors, EMG sensors, LFP sensors and/or implantable sensors.

**3.** An apparatus (1) in accordance with claim 1 or claim 2, wherein the stimulation unit (11) is non-invasive and is configured such that it generates stimuli from the group of acoustic, visual, tactile, vibratory, proprioceptive, thermal, olfactory and electrical transcutaneous stimuli.

**4.** An apparatus (1) in accordance with claim 1 or claim 2, wherein the stimulation unit (11) comprises one or more implantable electrodes for administering electrical stimuli.

**5.** An apparatus (1) in accordance with any one of the preceding claims, wherein the control and analysis unit (10) controls the stimulation unit (11) such that the intervals between mutually following stimuli are varied.

**6.** An apparatus (1) in accordance with any one of the preceding claims, wherein the control and analysis unit (10) is configured such that it determines a threshold value from the measured signals recorded before the administering of a stimulus and compares the probability determined from the measured signals recorded after the administering of the stimuli with which the phase distribution differs from an equal distribution with the threshold value to determine whether a pathological interaction between the brain areas is present.

**7.** An apparatus (1) in accordance with any one of the preceding claims, wherein the control and analysis unit (10) is configured such that it uses the Kuiper test to determine the probability with which the phase distribution differs from an equal distribution.

**8.** An apparatus (1) in accordance with any one of the preceding claims, wherein the control and analysis unit (10) is configured such that it detects at least one point in time at which the probability with which the phase distribution differs from an equal distribution is above a predefined threshold value; and/or integrates the probability with which the phase distribution differs from an equal distribution over a predefined time period; and/or determines the total duration of time periods in which the probability with which the phase distribution differs from an equal distribution is above a predefined threshold value.

## Revendications

1. Dispositif (1) pour l'examen d'une interaction pathologique entre différentes aires du cerveau, comprenant

   un module de stimulation (11) qui est configuré de telle sorte qu'il administre des stimuli identiques successivement à un patient, dans lequel les stimuli stimulent des neurones du patient dans les aires du cerveau à examiner,

   un module de mesure (12) pour l'enregistrement de signaux de mesure qui restituent une activité neuronale des neurones stimulés à une seule aire du cerveau, et

   un module de commande et d'analyse (10) pour la commande du module de stimulation et pour l'analyse des signaux de mesure, dans lequel le module de commande et d'analyse (10) est configuré de telle sorte qu'il commande le module de stimulation (11) de telle sorte que celui-ci administre les stimuli au patient, et

   transforme les signaux de mesure dans le plan complexe, examine la distribution des phases des signaux de mesure enregistrés par le module de mesure (12) en tant que réponse aux stimuli administrés au patient dans le plan complexe et détermine à l'aide de la distribution des phases des signaux de mesure qui restituent l'activité neuronale des neurones stimulés à la seule aire du cerveau en question la probabilité avec laquelle la distribution de phases se différencie d'une distribution égale pour déterminer si une interaction pathologique est présente entre les aires du cerveau.

2. Dispositif (1) selon la revendication 1, dans lequel le module de mesure (12) comprend des électrodes EEG, des capteurs MEG, des capteurs EMG, des capteurs LFP et/ou des capteurs implantables.

3. Dispositif (1) selon la revendication 1 ou 2, dans lequel le module de stimulation (11) est non invasif et est configuré de telle sorte qu'il génère des stimuli parmi le groupe des stimuli acoustiques, visuels, tactiles, vibratoires, proprioceptifs, thermiques, olfactifs et électriques transcutanés.

4. Dispositif (1) selon la revendication 1 ou 2, dans lequel le module de stimulation (11) comprend une ou plusieurs électrodes implantables pour l'administration de stimuli électriques.

5. Dispositif (1) selon une des revendications précédentes, dans lequel le module de commande et d'analyse (10) commande le module de stimulation (11) de telle sorte que les écarts entre des stimuli successifs sont variés.

6. Dispositif (1) selon une des revendications précédentes, dans lequel le module de commande et d'analyse (10) est configuré de telle sorte qu'il détermine à partir des signaux de mesure enregistrés avant l'administration d'un stimulus une valeur seuil et compare la probabilité déterminée à partir des signaux de mesure enregistrés après l'administration du stimulus avec laquelle la distribution de phases se différencie d'une distribution égale à la valeur seuil pour déterminer si une interaction pathologique est présente entre les aires du cerveau.

7. Dispositif (1) selon une des revendications précédentes, dans lequel le module de commande et d'analyse (10) est configuré de telle sorte qu'il utilise le test de Kuiper pour la détermination de la probabilité avec laquelle la distribution de phases se différencie d'une distribution égale.

8. Dispositif (1) selon une des revendications précédentes, dans lequel le module de commande et d'analyse (10) est configuré de telle sorte qu'il détecte au moins un instant auquel la probabilité avec laquelle la distribution de phases se différencie d'une distribution égale se situe au-dessus d'une valeur seuil prédéfinie, et/ou

   intègre la probabilité avec laquelle la distribution de phases se différencie d'une distribution égale sur une période prédéfinie, et/ou

   détermine la durée totale de périodes au cours desquelles la probabilité avec laquelle la distribution de phases se différencie d'une distribution égale se situe au-dessus d'une valeur seuil prédéfinie.

## Fig.1

## Fig.2

Fig.3A

Fig.3B

Fig.3C

Fig.3D

Fig.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120078323 A1

- US 20050273017 A1

**In der Beschreibung aufgeführte Nicht-Patentliteratur**

- **VON C. HAMMOND ; H. BERGMAN ; P. BROWN.** Pathological synchronization in Parkinson's disease: networks, models and treatments. *Trends Neurosci.,* 2007, vol. 30, 357-364
- **VON N. WEISZ ; S. MORATTI ; M. MEINZER ; K. DOHRMANN ; T. ELBERT.** Tinnitus Perception and Distress Is Related to Abnormal Spontaneous Brain Activity as Measured by Magnetoencephalography. *PLoS Med,* 2005, vol. 2 (6), 546-553
- **VON J. J. SCHULMAN ; R. CANCRO ; S. LOWE ; F. LU ; K. D. WALTON ; R. R. LLINÁS.** Imaging of Thalamocortical Dysrhythmia in Neuropsychiatry. *Front. Hum. Neurosci.,* 2011, vol. 5, 69
- **VON N. E. HUANG ; Z. SHEN ; S. R. LONG ; M. C. WU ; H. H. SHIH ; Q. ZHENG ; N.-C. YEN ; C. C. TUNG ; H. H. LIU.** The empirical mode decomposition and the Hilbert spectrum for nonlinear and non-stationary time series analysis. *Proc. R. Soc. A: Math. Phys. Eng. Sci.,* 1998, vol. 454, 903-995
- **VON W. HUANG ; Z. SHEN ; N. E. HUANG ; Y. C. FUNG.** Engineering analysis of biological variables: An example of blood pressure over 1 day. *Proc. Nat. Acad. Sci. USA,* 1998, vol. 95, 4816-4821
- **VON N. WEISZ ; S. MORETTI ; M. MEINZER ; K. DOHRMANN ; T. ELBERT.** Tinnitus Perception and Distress Is Related to Abnormal Spontaneous Brain Activity as Measured by Magnetoencephalography. *PLoS Med,* 2005, vol. 2 (6), 546-553
- **VON J. J. SCHULMAN ; R. CANCRO ; S. LOWE ; F. LU ; K. D. WALTON ; R. R. LLINÄS.** Imaging of Thalamocortical Dysrhythmia in Neuropsychiatry. *Front. Hum. Neurosci,* 2011, vol. 5, 69
- **VON G. D. DAWSON.** A summation technique for the detection of small evoked potentials. *Electroencephalogr. Clin. Neurophysiol.,* 1954, vol. 44, 153-154

- **VON M. HÄMÄLÄINEN ; R. HARI ; R. J. ILMONIEMI ; J. KNUUTILA ; O. V. LOUNASMAA.** Magnetoencephalography: Theory, instrumentation, and applications to noninvasive studies of the working human brain. *Rev. Mod. Phys.,* 1993, vol. 65, 413-497
- **VON W. SCHLEE ; N. MUELLER ; T. HARTMANN ; J. KEIL ; I. LORENZ ; N. WEISZ.** Mapping cortical hubs in tinnitus. *BMC Biol.,* 2009, vol. 7, 80
- **VON N. WEISZ ; S. MORATTI ; M. MEINZER ; K. DOHRMANN ; T. ELBERT.** Tinnitus Perception and Distress Is Related to Abnormal Spontaneous Brain Activity as Measured by Magnetoencephalography. *PLoS Med,* 2005, vol. 2 (6), 546-553
- **VON N. H. KUIPER.** Tests concerning random points on a circle. *Proceedings of the Koninklijke Nederlandse Akademie van Wetenschappen, Series A,* 1960, vol. 63, 38-47
- **VON E. BATSCHELET.** Circular Statistics in Biology. Academic Press, 1981
- **VON D. BILECEN ; K. SCHEFFLER ; N. SCHMID ; K. TSCHOPP ; J. SEELIG.** Tonotopic organization of the human auditory cortex as detected by BOLD-FMRI. *Hearing Research,* 1998, vol. 126, 19-27
- **VON D. R. M. LANGERS ; W. H. BACKES ; P. VAN DIJK.** Representation of lateralization and tonotopy in primary versus secondary human auditory cortex. *NeuroImage,* 2007, vol. 34, 264-273
- **VON W. MÜHLNICKEL ; T. ELBERT ; E. TAUB ; H. FLOR.** Reorganization of auditory cortex in tinnitus. *Proc. Natl. Acad. Sci. USA,* 1998, vol. 95, 10340-10343
- **VON B. A. WANDELL ; S. O. DUMOULIN ; A. A. BREWER.** Visual Field Maps in Human Cortex. *Neuron,* Oktober 2007, vol. 56, 366-383
- **A. BENNINGHOFF et al.** Lehrbuch der Anatomie des Menschen. Dargestellt unter Bevorzugung funktioneller Zusammenhänge. 3. Bd. Nervensystem, Haut und Sinnesorgane. *Urban und Schwarzenberg,* 1964